# EUROPEAN PATENT SPECIFICATION

(11) **EP 2 083 951 B1**
(45) Date of publication and mention of the grant of the patent: **08.08.2012**
(21) Application number: 07823951.4
(22) Date of filing: 28.09.2007
(51) Int. Cl.: B01L 3/14, B01L 3/00

(54) **SAMPLING TUBE**
PROBENRÖHRCHEN
TUBE DE PRÉLÈVEMENT

(30) Priority: 28.09.2006 GB 0619101; 06.07.2007 GB 0713112
(43) Date of publication of application: 05.08.2009
(73) Proprietor: Sterilin Limited, Newport NP11 3EF (GB)
(72) Inventor: ADAMS, Rachel, Stone, Staffordshire ST15 OSA (GB); NIGEL, James, Stone, Staffordshire ST15 OSA (GB)
(74) Representative: Teasdale, Nicola Joanne
(86) International application number: PCT/GB2007/003690
(87) International publication number: WO 2008/038012

(56) References cited:
- WO-A-02/28733
- US-A- 1 630 787
- US-A- 1 633 022
- US-A- 4 150 089
- US-A- 5 084 240
- US-A1- 2002 164 273

## Description

The present invention relates to an improved sampling tube, particularly but not exclusively a fluid sampling tube.

Urine analysis is a non-invasive way for initial patient health screening and is carried out in vast numbers in laboratories throughout the world. Current methodology is very labour intensive and time consuming and involves a number of manual steps such as centrifugation, re-suspension and microscopy. Automation of urine analysis is now being introduced into laboratories to dramatically increase throughput whilst also reducing the analytical variables to provide accurate, quantitative results. Automation allows a days work to be processed in a matter of hours thereby freeing up valuable clinician hours. Urine samples can be introduced into the machine without the requirement for any pre-treatment and each urine sample is treated in exactly the same way resulting in total standardisation of the methodology. The automated urinalysis machines are used as a negative screen on samples; all negatives are disposed of whilst those providing a positive result are subsequently cultured. This may lead to a possible reduction in cultures by 50%.

Two types of urinalysis machines are predominantly in use throughout Europe. The UF series, manufactured by Sysmex which incorporates the UF100™, UF1000 and UF1000i operate by flow cytometry. The machines aspirate approximately 800µl of urine and perform the analysis. Cells, bacteria and casts are measured by scattered light and the use of dyes and then categorised according to size, shape, volume and staining characteristics. The results are displayed as a scattergram on screen and problematic specimens are often investigated using manual microscopy. The IQ200 Sprint™ machine operates by digital imagery and performs microscopy on unspun samples. The equipment uses Auto-Particle Recognition (APR) to identify and quantitate twelve formed elements. Images can be viewed on demand at the workstation monitor.

The equipment racking only takes a 10ml tube whereas the primary collection device for urine in the UK and many parts of mainland Europe is a 30ml universal container or larger which, in the laboratory, has to be manually decanted into a suitable 10 ml tube for automated analysis. The machine processes the samples in sequences of ten (each rack holding ten different patient samples) where a probe enters the tube and obtains the required volume sample. This is done ten times for each rack, the probe being rinsed by the machine between each sampling to endeavour to ensure "clean entry". Due to the automated sampling process, the manufacturers cannot guarantee that there is no carryover between samples, that is, bacterial transfer from one sample into subsequent patient samples. Therefore, in the laboratory, all samples are divided between two containers prior to processing with only sample from the clean container, which has not been processed by the machine, being used for culture analysis.

It is clear that this dual sampling procedure has many drawbacks for the laboratory, including the time consuming process of decanting urine and dual bar coding of the separated samples. It is also costly due to the requirement for two separate containers. Furthermore, decanting urine samples is a hazardous process and is open to errors due to the number of samples handled.

Whilst a number of prior art documents disclose tubes for receiving and storing fluids such as urine, for example, GB 1234044, GB 2404735, US 5897840, US 4473530, US 3774455 and GB 1428034, none of the documents disclose urine analysis sampling tubes which address the problems outlined above and which are suitable for use with automated sampling equipment.

For example in WO 02/28733 (Cryo-Cell International Inc.) there is described a segmented vial assembly and related storage and retrieval method in which the segmented cryogenic container assembly includes a vial or ampule having a storage chamber for storing biological specimens at lower temperature. The vial or ampule contains at least one partition member subdividing the chamber into a plurality of intercommunicating storage compartments.

Likewise In US 5,084,240 (Cirrus Diagnostics Inc.) there is described a centrifuge vessel for automated solid-phase immunoassay in which the centrifuge vessel comprises a centre tube and an outer waste chamber such that biomaterial held within the centre tube is capable of both binding specific analytes in test samples.

It is an object of the present invention to provide a sampling tube that aims to overcome the abovementioned drawbacks, removing the need for separation of a sample from the first container into a second (or more) further containers but still allowing its use in automated urinalysis machines.

Accordingly, the present invention provides (500) suitable for use in automated sampling machines comprising a sterile or aseptically manufactured tube having an open end, a closed end and an outer external wall, the tube further comprising a primary compartment (508) and at least one secondary compartment (518), wherein:
the primary compartment and the at least one secondary compartment are separated by at least one internal dividing wall (516) which is shorter in length than the outer external wall (522) and does not extend fully to the open end of the tube; and characterised in that:
   the at least two compartments are adapted to enable fluids contained therewithin to be kept separate but allow fluid communication between the compartments if desired; and wherein
   at least part of the tube is substantially circular in cross-section and at least part of the tube comprises a substantially continuous cross-section: and
   wherein the at least one dividing wall is provided between the two compartments with a gap above the dividing wall up to the open end of the tube; and wherein
   the tube is adapted to keep fluid in the at least two compartments separate when the tube is in the substantially vertical position but also fluid to flow between the compartments upon tilting of the tube; and
   wherein the dividing wall separates the first compartment and the second compartment is provided at or towards the periphery of the tube away from the centre of the primary compartment; and
   wherein the sampling tube comprises a neck region and a main body portion and wherein the at least one secondary compartment is located within the neck region of the sample tube towards the open end of the tube; and
   wherein the at least one secondary compartment comprises a cup located in the neck region of the sample tube towards the open end of the tube such that when the sampling tube is inverted fluid present in the sampling tube flows over the internal dividing wal (516) Into the second compartment (518) of the cup, which forms a moat area within the neck of the sampling tube; and
   wherein the main body portion of the sampling tube comprises a finger-like projection of substantially constant external profile.

The sampling tube is particularly suitable for the collection and analysis of urine. However, it is to be appreciated that it may be used for the collection and analysis of other types of fluid, such as blood.

The sampling tube preferably further comprises a detachable closure means for attaching to the open end of the tube for providing a leakproof container, preferably the closure means comprises, for example but not limited to, a screw cap. A sealing ring may be provided between the cap and the tube.

At least two compartments are preferably adapted to enable the fluid contained therewithin to be kept separate but preferably, allow fluid communication between the compartments when desired. For example, fluid in the two compartments is preferably kept separate when the tube is in the substantially vertical position but, fluid is able to flow between the compartments upon tilting of the tube. This may be achieved by providing at least one dividing wall between the two compartments with a gap provided above the wall up to the open end of the tube whereb, upon placement of the cap, the tube may be inverted to allow fluid flow from the primary compartment to the secondary compartment.

Preferably, the size and positioning of the secondary compartment relative to the primary compartment is such that, upon inversion of the tube, a volume of fluid enters and is collected in the secondary compartment from the primary compartment.

Preferably, at least part of the tube is circular in cross-section. Preferably, the secondary compartment extends longitudinally from the open end of the tube parallel to the primary compartment that forms the main body of the tube. The secondary compartment is preferably provided at or towards the periphery of the tube, that is, away from the centre of the primary compartment. For example, the dividing wall forms a chord of the circle that does not pass through the centre of the circle. The secondary compartment may extend the length of the primary compartment or, more preferably, extends only partially along its length.

In one embodiment, the secondary compartment and primary compartment together form a circular cross-section with the portion of the tube comprising only primary compartment forming a truncated circle in cross-section. However, it is preferable for the outer profile of the tube to resemble a conventional cylindrical tube. Therefore, according to another embodiment of the present invention, the portion of the tube comprising only the primary compartment is provided with ribs extending from the outer wall thereof below the secondary compartment, the outer ends of the ribs mimicking the profile of a standard tube. However, it is to be appreciated that other suitable means may be provided to complete the missing segment of the circular cross-section.

The sampling tube according to the present invention is preferably formed of a plastics material, more preferably general purpose crystal polystyrene or polypropylene.

Appropriate markings may be provided on the tube, such as a maximum fill line to identify the level of fluid that should be collected in the primary and/or secondary compartment.

The relative dimensions of the compartments making up the aseptically produced tube may be adjusted to suit the intended application. Preferably, the dimension of the tube are 17mm x 100mm.

Any suitable process may be employed for producing the compartmentalized tube, such as ultrasonic welding.

In a further embodiment of the preset invention the neck region of the open end of the tube is important and comprises a neck region and a main body region, wherein the neck region is wider in diameter than the main body region.

In a preferred embodiment, the at least one secondary compartment is located in the neck region of the sample tube towards the open end of the tube. The at least one secondary compartment may preferably comprise a cup located in the neck region of the sample tube towards the open end of the tube.

The volume of the at least one secondary compartment is preferably between 300mm³ and 1000mm³. The diameter of the neck region preferably comprises 12mm to 28mm, more preferably 14mm to 17mm.

At the opposite end of the tube, that is the end of the sampling tube remote from the open end, the tube forms a finger-like projection.

This end of the sampling tube remote from the open end is either rounded or conical or flat.

In yet a further embodiment of the sampling tube according to the present invention the secondary compartment is further divided and comprises between 2 and 4 compartments.

For any of the sampling tubes described the tube preferably comprises a plastics material, preferably either polystyrene or polypropylene.

Furthermore, the sampling tubes as described may be used for the collection and sampling of bodily fluids, more preferably for the collection and sampling of urine.

For a better understanding of the present invention and to show more clearly how it may be carried into effect reference will now be made by way of example only to accompanying drawings in which:
Figure 1 is a front plan view of a sampling tube according to a first embodiment of the present invention;
Figure 2 is a longitudinal sectional view of the sampling tube shown in Figure 1;
Figure 3 is a top plan view of the sampling tube shown in Figure 1;
Figure 4 is a front plan view of a sampling tube according to a second embodiment of the present invention;
Figure 5 is a side plan view of the sampling tube shown in Figure 4;
Figure 6 is a longitudinal sectional side view of the sampling tube shown in Figure 4;
Figure 7 is a top plan view of the sampling tube shown in Figure 4;
Figure 8 is a cross-sectional view along line A-A shown in Figure 6;
Figure 9 is a front plan view of sampling tube according to a third embodiment of the present invention;
Figure 10 is a rear plan view of the sampling tube shown in Figure 9;
Figure 11 is a longitudinal sectional side view of the tube shown in Figure 9;
Figure 12 is a top plan view of the tube shown in Figure 9;
Figure 13 is a cross-sectional view along line B-B shown in Figure 11;
Figures 14 a to d are full and partial views of a sampling tube according to a fourth embodiment of the present invention;
Figures 15a to d are full and partial views of a sampling tube according to a fifth embodiment of the present invention;
Figures 16 a to c are respectively a front plan view, longitudinal sectional view and top plan view of a sampling tube according to a sixth embodiment of the present invention; and
Figures 17 a to c are respectively a front plan view, longitudinal sectional view and top plan view of a sampling tube according to a seventh embodiment of the present invention.
Figures 18 a to e are respectively
   a front plan view,
   a longitudinal sectional view and,
   a top plan views of a sampling tube according to an eighth embodiment of the present invention with one, two and four compartments respectively.
Figures 19 a to d are respectively
   a front plan view,
   a longitudinal sectional view,
   an enlarged cross-sectional view of the neck region and,
   a top plan view of a sampling tube according to a ninth embodiment of the present invention.

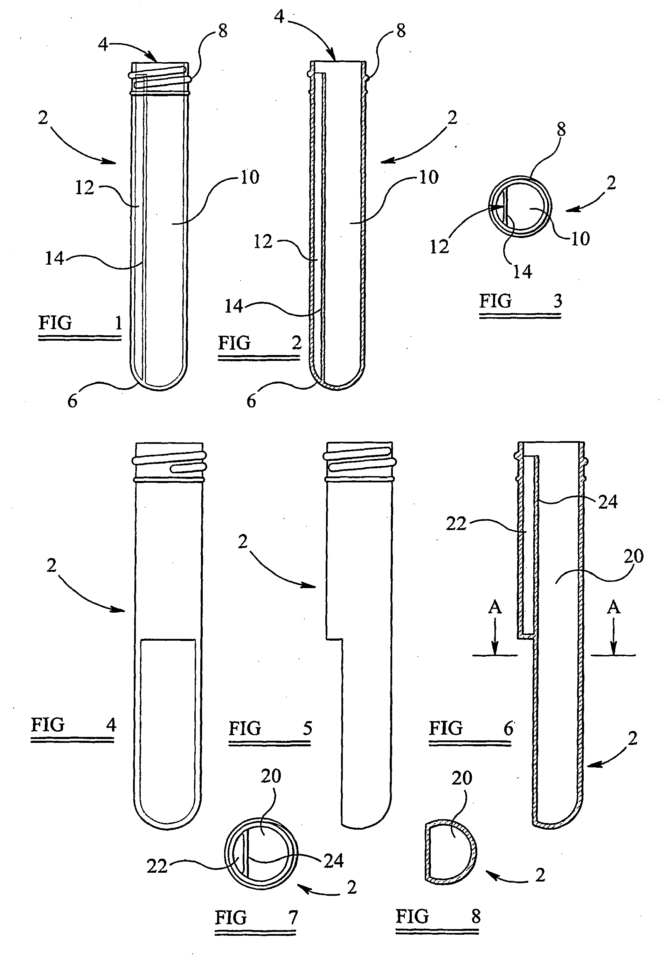

Referring to Figures 1 to 3 of the accompanying drawings, a sterile or aseptically produced sampling tube 2 according to one embodiment of the present invention is illustrated. The finger-like tube is open at one end 4 and closed at the other end 6 and is provided with screw threads 8 around its upper end for receiving a screw cap (not shown). The tube is 17mm x 100mm. The interior of the tube is divided into two compartments, a primary or main compartment 10 and a secondary compartment 12 located at one side of the tube. An internal dividing wall 14 separates the two compartments, the dividing wall extending from the closed end of the tube to just before the upper end of the tube. A fill level line (not shown) is provided on the tube to indicate the level of fluid that should be collected in the tube. This may be provided directly on the tube or, for example, on a label that is then stuck onto the tube.

The sampling tube according to the present invention enables two discrete samples of fluid, such as urine, to be retained within a single sampling tube. A patient delivers the requested sample into the tube up to the fill level and the sample is sealed by means of the screw cap. The sample is then delivered to a hospital laboratory for analysis where a technician inverts the tube to fill the secondary compartment with fluid from the main compartment prior to insertion in the racking of an automated analysis machine. This enables a specific volume of fluid to be retained in the secondary compartment separate to the fluid in the main compartment. The fluid in the main compartment is then subjected to automated analysis by the insertion of a probe. This does not come into contact with the fluid of the secondary compartment due to the dividing wall therebetween. Furthermore, the positioning of the secondary compartment towards the periphery of the tube (that is, away from the centre of the main compartment) ensures that the probe cannot contact the dividing wall or secondary compartment which could result in damage to the probe. The clean sample in the secondary compartment may then be used for culture if a positive result is found on testing the fluid in the primary compartment.

Figures 4 to 8 of the accompanying drawings illustrate a second embodiment of the present invention. Identical features to those already discussed in relation to Figures 1 to 3 are given the same reference numerals and only the differences will be discussed in detail. The sampling tube again has a main compartment 20 and a secondary compartment 22 but the main compartment is in the form of a truncated cylinder with the secondary compartment being in the form of a segment that completes the cylinder in the upper region of the tube. In this manner, the secondary tube is again located at the periphery of the main compartment but extends only part way along the length thereof. The dividing wall 24 between the main and secondary compartments falls short of the upper outer walls of the tube to enable fluid communication between the two compartments when the cap is screwed on and the tube is tilted/inverted.

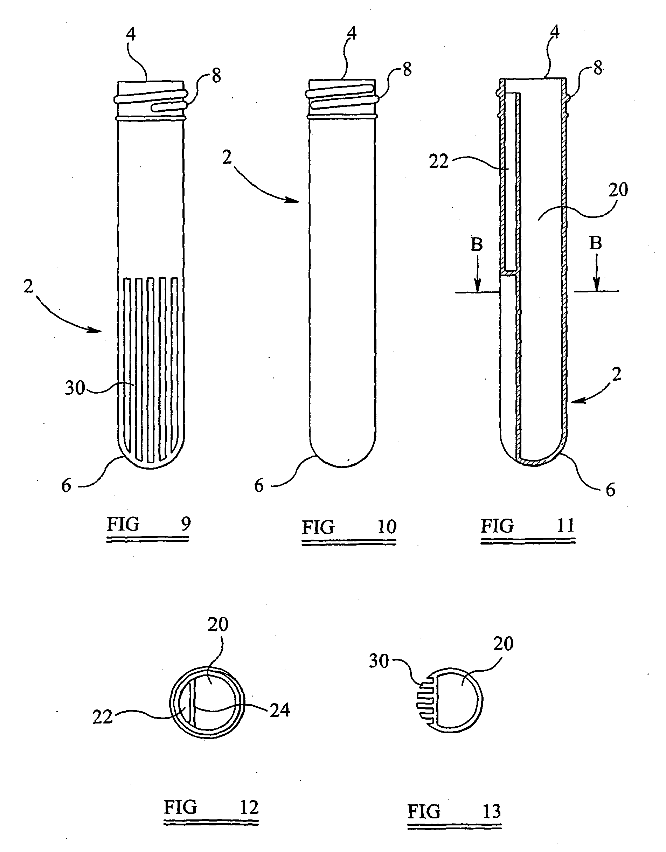

Figures 9 to 13 illustrate another embodiment of the present invention. This embodiment is very similar to that shown in Figures 4 to 8 and therefore the same reference numerals are used except ribs 30 are provided in the indented region of the tube that is formed below the secondary compartment to provide the sampling tube with an outer profile that substantially mimics the profile of a conventional test tube. This is not only aesthetically pleasing but should provide a more stable tube when it is held within the racking of an automated analysis machine.

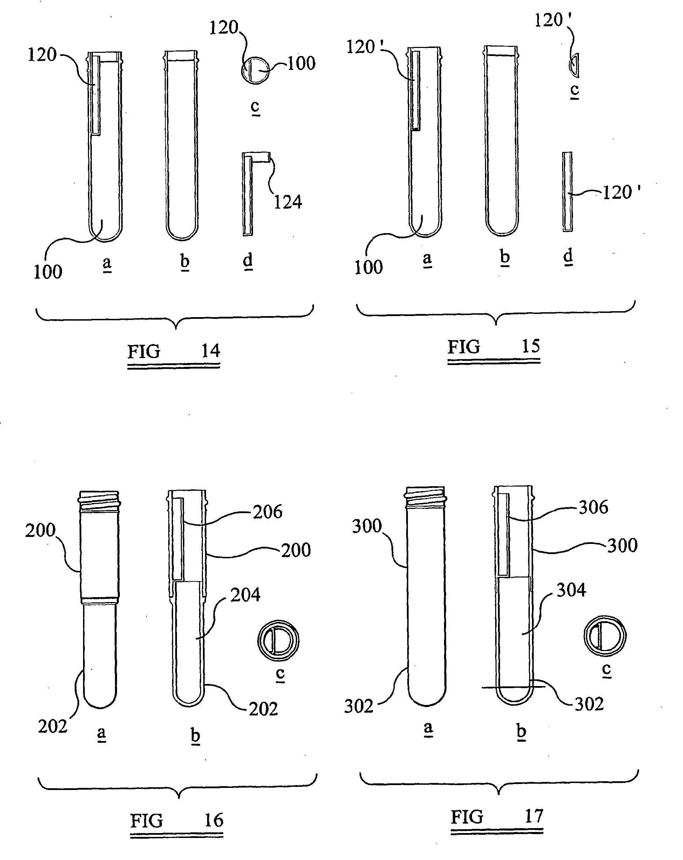

The two compartments may be welded together, for example using ultrasonic welding or secured together by other means. Figures 14 a to d illustrate a sampling tube having a main compartment 100 and secondary compartment 120 wherein the compartment 120 is secured to the periphery of the main compartment by means of a ring clip 124. Figures 15 a to d illustrates a similar tube but having the secondary segment 120' ultrasonically welded to the side of the main compartment 100. The tube may also be formed of an upper and lower portion (200, 202 and 300 and 302 in Figures 16 and 17 respectively) that are welded together, the secondary compartment 206, 306 being provided in the upper portion.

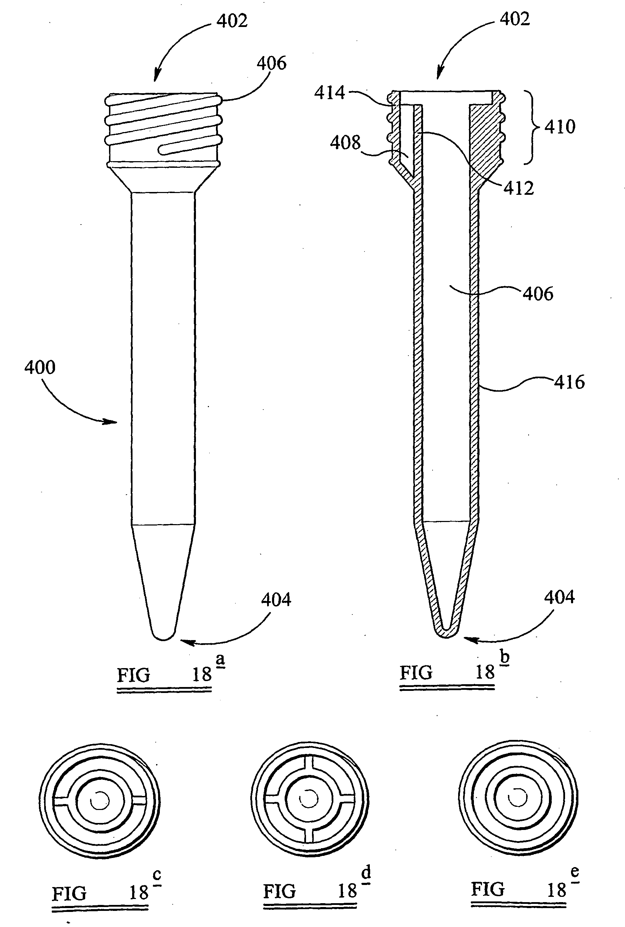

Figures 18 a, b, c and d of the accompanying drawings again illustrate an aseptically produced sampling tube 400 according to an eighth embodiment of the present invention. The finger-like tube is open at one end 402, and closed at the other end 404 and is provided with screw threads 406 around its upper end for receiving a screw cap (not shown) as with the previously described embodiments. It will be appreciated that other closure means other than a screw cap may be employed in the embodiments of the present invention so long as the closure means prevents the loss of fluid from the sampling tube, and further allows the sampling tube to be inverted, thereby allowing the fluid to be decanted from the first to the second compartment of the sampling tube.

Figure 18 b illustrates a longitudinal sectional view of the sampling tube 400, and depicts the two compartments of the tube, a first compartment 406 and a second compartment 408. The second compartment is again located in the neck region 410 of the sampling tube. An internal dividing wall 412 separates the first and second compartment. The dividing wall 412 is somewhat shorter in length than the outer wall of the sampling tube 414. Consequently, when the sampling tube is inverted with a closed end, fluid flows over the internal dividing wall 412 into the second compartment prior to insertion of the tube in the racking of an automated analysis machine. The second compartment 408 can comprise a single compartment as is Figure 18e, or may comprises separate compartments as can be seen in Figures 18 c and 18d.

In the sampling tube 400 according to the eighth embodiment of the present invention, it can be seen that the neck region of the tube 410 is wider than the main body portion of the tube 416. The widened neck region 410 of the tube provides a larger and hence more easily accessible area for insertion of a sampling probe of the analysis machine. Consequently, the sampling tubes are less likely to be damaged during insertion of the probe into the neck region of the tube. The larger neck region of the tube also provides a convenient location for placing patient details and a bar code reader as required. The length of the tube is preferably between 112mm and 120mm, more preferably 112mm to 115mm. The length of the tube to the base of the neck region is preferably between 95mm and 110mm, even more preferably between 95mm and 106mm and most preferably 100mm.

The diameter of the widened region is preferably between 15mm and 25mm, more preferably between 16mm and 22mm and most preferably between 16mm and 18.5mm. It will be appreciated that the size and shape of the neck region of the sampling tube has to be selected such that when a number of sampling tubes are placed within the compartments of a sampling tube rack, preferably, each of the compartments are able to be occupied by sampling tubes with no compartments vacated to allow additional space for the sampling tubes.

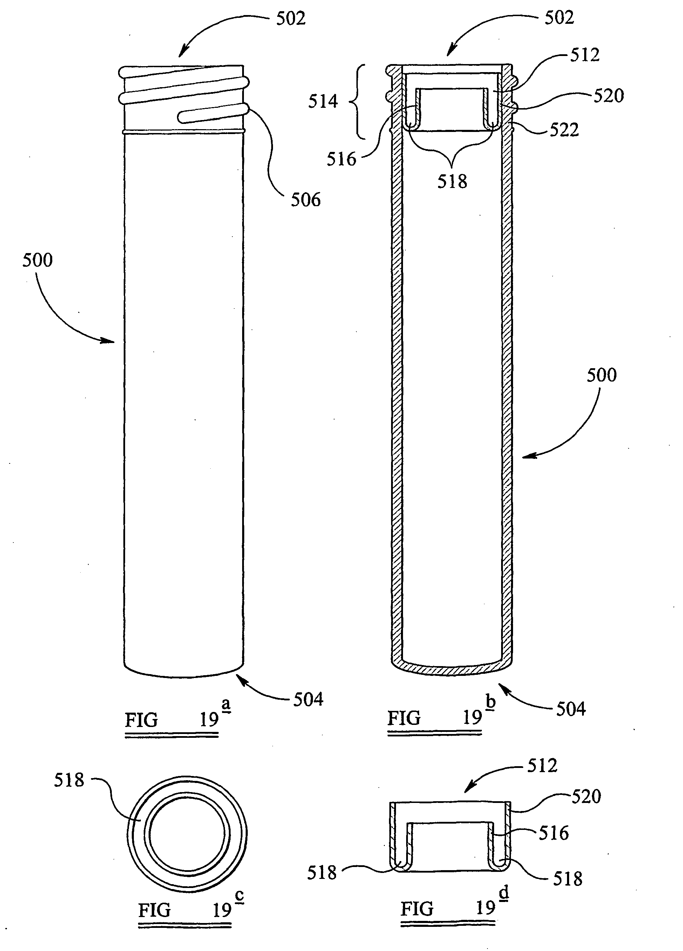

Figures 19 a, b, c and d of the drawings illustrate a further sampling tube 500 according to a ninth aspect of the present invention. The sampling tube has an open end 502, and a closed end 504. Whilst the tube is depicted with a rounded closed end as in embodiments 1 to 7, previously described above, it will be appreciated that this ninth embodiment and embodiments 1 to 7 may equally be constructed with a finger-like closed end region as in embodiment 8 and Figure 18 a and b. As with the other embodiments, this ninth embodiment sampling tube is exemplified with screw threads 506, fitted around the upper open end of the tube for receiving a screw cap (not shown). Other closure means may however be employed.

Figure 19 b illustrates a longitudinal sectional view of the sampling tube 500. The sampling tube comprises a first compartment 508, which forms the main body of the sampling tube. However, this ninth embodiment of the present invention comprises a receptacle or cup 512 which is located in the neck region 514 of the sampling tubes preferably 10mm from the opening. An internal dividing wall 516 of the cup, separates the first compartment 508 from the second compartment 518. The dividing wall 516 is again somewhat shorter in length than the outer wall 520 of the cup, and the outer wall 522 of the sampling tube. Therefore, when the screw cap is fitted and the sampling tube is inverted, fluid present in the sampling tube flows over the internal dividing wall 516 in the second compartment 518 of the cup, which forms a moat area within the neck of the sampling tube. In this way, a suitable amount of fluid can be effectively separated from the main body of the sampling tube. Therefore, the separated fluid is not contaminated by insertion of a probe into the main sampling tube contents when the tube is placed in an analysis machine. The sampling tube and cup are preferably comprised of a plastics material, preferably polystyrene or polypropylene. The distance between the outer walls of the cup is preferably between 12mm and 20mm, most preferably 15mm. The diameter between the internal dividing walls of the cup is preferably between 8mm and 12mm, most preferably 9mm.

In a preferred embodiment of the sampling tube 500 according to the ninth aspect of the present invention, the cup is preferably formed as a separate piece from the sampling tube and is fixed in place in the neck region of the tube via a suitable means for example but not limited to, a clip means, ultra-sonic welding or via an interference push-fit mechanism. The cup preferably has a volume of between 300mm³ and 1000mm³. In a preferred embodiment of the invention the sampling tube comprises a neck region and a main body region. Preferably the neck region is wider in diameter than the main body region such that the diameter of the neck region is between 12m and 28 mm, more preferably 14mm to 17mm.

Therefore, the aseptically produced sampling tube is prepared according to the nine embodiments of the present invention clearly have a number of benefits over a conventional 30ml urine containers that are currently used to collect urine. Firstly, there is a huge time-saving achieved as there is no requirement to manually decant urine into a secondary tube for culture analysis nor re-apply an appropriate barcode. This feature also significantly reduces the possibility of errors occurring with patient samples. The tube is also cost effective to produce as only one tube is required and is user friendly as it is easy to fill. It also eliminates the potentially hazardous process of decanting samples by laboratory staff.

## Claims

1. A sampling tube (500) suitable for use in automated sampling machines comprising a sterile or aseptically manufactured tube having an open end, a closed end and an outer external wall, the tube further comprising a primary compartment (508) and at least one secondary compartment (518), wherein:
the primary compartment and the at least one secondary compartment are separated by at least one internal dividing wall (516) which is shorter in length than the outer external wall (522) and does not extend fully to the open end of the tube; and **characterised in that**;
the at least two compartments are adapted to enable fluids contained therewithin to be kept separate but allow fluid communication between the compartments if desired; and wherein
at least part of the tube is substantially circular in cross-section and at least part of the tube comprises a substantially continuous cross-section: and
wherein the at least one dividing wall is provided between the two compartments with a gap above the dividing wall up to the open end of the tube; and wherein
the tube is adapted to keep fluid in the at least two compartments separate when the tube is In the substantially vertical position but also fluid to flow between the compartments upon tilting of the tube; and
wherein the dividing wall separates the first compartment and the second compartment is provided at or towards the periphery of the tube away from the centre of the primary compartment; and
wherein the sampling tube comprises a neck region and a main body portion and wherein the at least one secondary compartment is located within the neck region of the sample tube towards the open end of the tube; and
wherein the at least one secondary compartment comprises a cup located in the neck region of the sample tube towards the open end of the tube such that when the sampling tube is inverted fluid present in the sampling tube flows over the internal dividing wal (516) into the second compartment (518) of the cup, which forms a moat area within the neck of the sampling tube; and
wherein the main body portion of the sampling tube comprises a finger-like projection of substantially constant external profile.

2. A sampling tube as claimed in claim 1 further comprising a detachable cap for attaching to the open end of the tube for providing a leak proof container such that upon placement of the cap the tube may be inverted or tilted to allow fluid flow from the primary compartment to the secondary compartment.

3. A sampling tube as claimed in claim 2 wherein the cap is in the form of a screw cap and a sealing ring is provided between the cap and the tube.

4. A sampling tube as claimed in claim 1 wherein the size and positioning of the secondary compartment relative to the primary compartment is such that, upon inversion of the tube, a volume of fluid enters and is collected In the secondary compartment from the primary compartment.

5. A sampling tube as claimed in any one of the preceding claims wherein at least part of the tube is circular in cross-section and the secondary compartment extends longitudinally from the open end of the tube, parallel with the primary compartment that forms the main body of the tube.

6. A sampling tube as claimed in claim 5 wherein the secondary compartment is provided at or towards the periphery of the tube, that is, away from the centre of the primary compartment.

7. A sampling tube as claimed in any one of the preceding claims wherein the secondary compartment extends either the length of the primary compartment or extends only partially along its length.

8. A sampling tube as claimed in any one of claims 5 to 7 wherein the at least one secondary compartment and primary compartment together form a circular cross-section and wherein the portion of the tube comprising only the primary compartment comprises a truncated circle in cross-section.

9. A sampling tube as claimed in claim 8 wherein means is provided to provide the tube with an outer profile that resembles a conventional cylindrical tube.

10. A sampling tube as claimed in claim 9 wherein the portion of the tube comprising only the primary compartment is provided with ribs extending from the outer wall thereof below the secondary compartment, the outer ends of the ribs mimicking the profile of a conventional cylindrical tube.

11. A sampling tube according to any of the preceding claims wherein the sampling tube further comprises a neck region and a main body region and wherein the neck region is wider in diameter than the main body region.

12. A sampling tube according to any of the preceding claims wherein the secondary compartment is further divided and comprises between 2 and 4 compartments.

13. Use of a sampling tube as claimed in any one of the preceding claims for the collection and sampling of bodily fluids such as urine.

## Patentansprüche

1. Probenröhrchen (500) zur Verwendung in automatisierten Probenmaschinen, die ein steriles und aseptisch hergestelltes Röhrchen mit einem offenen Ende, einem geschlossenen Ende und einer externen Außenwand umfasst, wobei das Röhrchen ferner eine primäre Kammer (508) und wenigstens eine sekundäre Kammer (518) beinhaltet, wobei:
die primäre Kammer und die wenigstens eine sekundäre Kammer durch wenigstens eine interne Trennwand (516) getrennt sind, deren Länge geringer ist als die der externen Außenwand (522) und die nicht ganz bis zum offenen Ende des Röhrchens verläuft; und **dadurch gekennzeichnet, dass**:
die wenigstens zwei Kammern so gestaltet sind, dass darin enthaltene Fluide separat gehalten werden können, aber eine Fluidverbindung bei Bedarf zwischen den Kammern zugelassen wird; und wobei
wenigstens ein Teil des Röhrchens im Wesentlichen kreisförmig im Querschnitt ist und wenigstens ein Teil des Röhrchens einen im Wesentlichen kontinuierlichen Querschnitt hat; und
wobei die wenigstens eine Trennwand zwischen den beiden Kammern mit einer Lücke über der Trennwand bis zum offenen Ende des Röhrchens versehen ist; und wobei
das Röhrchen so gestaltet ist, dass es Fluid in den wenigstens zwei Kammern separat hält, wenn das Röhrchen in der im Wesentlichen vertikalen Lage ist, aber beim Kippen des Röhrchens auch Fluid zwischen den Kammern fließen lässt; und
wobei die die erste Kammer und die zweite Kammer trennende Trennwand an oder nahe der Peripherie des Röhrchens fern von der Mitte der primären Kammer vorgesehen ist; und
wobei das Probenröhrchen eine Halsregion und einen Hauptkörperabschnitt aufweist und wobei sich die wenigstens eine sekundäre Kammer in der Halsregion des Probenröhrchens nahe dem offenen Ende des Röhrchens befindet; und
wobei die wenigstens eine sekundäre Kammer einen Becher aufweist, der sich in der Halsregion des Probenröhrchens nahe dem offenen Ende des Röhrchens befindet, so dass, wenn das Probenröhrchen umgekehrt wird, in dem Probenröhrchen vorhandenes Fluid über die innere Trennwand (516) in die zweite Kammer (518) des Bechers fließt, einen Grabenbereich im Hals des Probenröhrchens bildend; und
wobei der Hauptkörperabschnitt des Probenröhrchens einen fingerartigen Vorsprung mit einem im Wesentlichen konstanten Außenprofil aufweist.

2. Probenröhrchen nach Anspruch 1, das ferner eine abnehmbare Verschlusskappe zum Anbringen am offenen Ende des Röhrchens umfasst, um einen lecksicheren Container zu bilden, so dass das Röhrchen nach dem Aufsetzen der Verschlusskappe umgekehrt oder gekippt werden kann, so dass Fluid von der primären Kammer in die sckundäre Kammer fließen kann.

3. Probenröhrchen nach Anspruch 2, wobei die Verschlusskappe in Form einer Schraubkappe vorliegt und ein Dichtungsring zwischen der Verschlusskappe und dem Röhrchen vorgesehen ist.

4. Probenröhrchen nach Anspruch 1, wobei Größe und Lage der sekundären Kammer relativ zur primären Kammer derart sind, dass nach dem Umkehren des Röhrchens ein Fluidvolumen aus der primären Kammer in die sekundäre Kammer eintritt und dort entnommen wird.

5. Probenröhrchen nach einem der vorherigen Ansprüche, wobei wenigstens ein Teil des Röhrchens kreisförmig im Querschnitt ist und die sekundäre Kammer longitudinal vom offenen Ende des Röhrchens parallel zur primären Kammer verläuft, die den Hauptkörper des Röhrchens bildet.

6. Probenröhrchen nach Anspruch 5, wobei die sekundäre Kammer an oder nahe der Peripherie des Röhrchens, d.h. fern von der Mitte der primären Kammer vorgesehen ist.

7. Probenröhrchen nach einem der vorherigen Ansprüche, wobei die sekundäre Kammer entweder über die Länge der primären Kammer oder nur teilweise über deren Länge verläuft.

8. Probenröhrchen nach einem der Ansprüche 5 bis 7, wobei die wenigstens eine sekundäre Kammer und die primäre Kammer zusammen einen kreisförmigen Querschnitt bilden und wobei der Abschnitt des Röhrchens, der nur die primäre Kammer umfasst, einen abgeschnittenen Kreis im Querschnitt umfasst.

9. Probenröhrchen nach Anspruch 8, wobei Mittel vorgesehen sind, um das Röhrchen mit einem Außenprofil zu versehen, das einem herkömmlichen zylindrischen Röhrchen ähnelt.

10. Probenröhrchen nach Anspruch 9, wobei der Abschnitt des Röhrchens, das nur die primäre Kammer umfasst, mit Rippen versehen ist, die sich von deren Außenwand unter der zweiten Kammer erstrecken, wobei die äußeren Enden der Rippen das Profil eines herkömmlichen zylindrischen Röhrchens nachahmen.

11. Probenröhrchen nach einem der vorherigen Ansprüche, wobei das Probenröhrchen ferner eine Halsregion und eine Hauptkörperregion umfasst und wobei die Halsregion einen breiteren Durchmesser hat als die Hauptkörperregion.

12. Probenröhrchen nach einem der vorherigen Ansprüche, wobei die sekundäre Kammer weiter unterteilt ist und zwischen 2 und 4 Kammern umfasst.

13. Verwendung eines Probenröhrchens nach einem der vorherigen Ansprüche zum Sammeln und Nehmen von Körperfluidproben wie z.B. Urin.

## Revendications

1. Tube de prélèvement (200), adapté pour être utilisé dans une machine d'échantillonnage automatique, comprenant un tube stérile ou à fabrication aseptique, comportant une extrémité ouverte, une extrémité fermée et une paroi externe extérieure, le tube comprenant en outre un compartiment primaire (508) et au moins un compartiment secondaire (518) ;
le compartiment primaire et le au moins un compartiment secondaire étant séparés par au moins une paroi de division interne (516) ayant une longueur inférieure à celle de la paroi externe extérieure (522) et ne s'étendant pas complètement vers l'extrémité ouverte du tube ; et **caractérisé en ce que** :
les au moins deux compartiments sont adaptés pour permettre la retenue dans un état séparé de fluides qui y sont contenus, mais pour permettre si nécessaire une communication de fluide entre les compartiments ; et dans lequel :
au moins une partie du tube une section transversale pratiquement circulaire, et au moins une partie du tube comprend une section transversale pratiquement continue ; et
dans lequel la au moins une paroi de division est agencée entre les deux compartiments, avec un espace au-dessus de la paroi de division, jusqu'à l'extrémité ouverte du tube ; et dans lequel :
le tube est adapté pour retenir le fluide dans les au moins deux compartiments dans un état séparé lorsque le tube se trouve dans une position pratiquement verticale, mais pour permettre l'écoulement du fluide entre les compartimentes lors de l'inclinaison du tube ; et
dans lequel la paroi de séparation séparant le premier compartiment et le deuxième compartiment est agencée au niveau ou en direction de la périphérie du tube, à l'écart du centre du compartiment primaire ; et
dans lequel le tube de prélèvement comprend une région de col et une partie de corps principal, le au moins un deuxième compartiment étant agencé dans la région de col du tube de prélèvement, en direction de l'extrémité ouverte du tube ; et
dans lequel le au moins un compartiment secondaire comprend un coupelle agencée dans la région de col du tube de prélèvement, en direction de l'extrémité ouverte du tube, de sorte que lorsque le tube de prélèvement est retourné, le fluide présent dans le tube de prélèvement s'écoule au-dessus de la paroi de division interne (516) dans le compartiment secondaire (516) de la coupelle, formant une zone de cavité dans le col du tube de prélèvement ;
dans lequel la partie de corps principal du tube de prélèvement comprend une saillie en forme de doigt, ayant un profil externe pratiquement constant.

2. Tube de prélèvement selon la revendication 1, comprenant en outre un bouchon détachable destiné à être fixé sur l'extrémité ouverte du tube pour fournir un récipient étanche aux fuites, de sorte que lors de la mise en place du bouchon, le tube peut être retourné ou incliné pour permettre l'écoulement du fluide du compartiment primaire vers le compartiment secondaire.

3. Tube de prélèvement selon la revendication 2, dans lequel le capuchon a la forme d'un bouchon à vis, une bague d'étanchéité étant agencée entre le bouchon et le tube.

4. Tube de prélèvement selon la revendication 1, dans lequel la taille et le positionnement du compartiment secondaire par rapport au compartiment primaire sont tels que lorsque le tube est retourné, un volume de fluide rentre dans le compartiment secondaire à partir du compartiment primaire et y est collecté.

5. Tube de prélèvement selon l'une quelconque des revendications précédentes, dans lequel au moins une partie du tube a une section transversale circulaire, le compartiment secondaire s'étendant longitudinalement à partir de l'extrémité ouverte du tube, de manière parallèle au compartiment primaire constituant le corps principal du tube.

6. Tube de prélèvement selon la revendication 5, dans lequel le compartiment secondaire est agencé au niveau ou en direction de la périphérie du tube, donc à l'écart du centre du compartiment primaire.

7. Tube de prélèvement selon l'une quelconque des revendications précédentes, dans lequel le compartiment secondaire s'étend le long de la longueur du compartiment primaire ou uniquement en partie le long de sa longueur.

8. Tube de prélèvement selon l'une quelconque des revendications 5 à 7, dans lequel le au moins un compartiment secondaire et le compartiment primaire forment ensemble une section transversale circulaire, la partie du tube comprenant uniquement le compartiment primaire ayant une section transversale en cercle tronqué.

9. Tube de prélèvement selon la revendication 8, comprenant un moyen pour conférer au tube un profil externe ressemblant à un tube cylindrique conventionnel.

10. Tube de prélèvement selon la revendication 9, dans lequel la partie du tube comprenant uniquement le compartiment primaire comporte des nervures s'étendant à partir de sa paroi externe, au-dessous du compartiment secondaire, les extrémités externes des nervures simulant le profil d'un tube cylindrique conventionnel.

11. Tube de prélèvement selon l'une quelconque des revendications précédentes, dans lequel le tube de prélèvement comprend en outre une région de col et une région de corps principal, la région de col ayant un diamètre supérieur à celui de la région de corps principal.

12. Tube de prélèvement selon l'une quelconque des revendications précédentes, dans lequel le compartiment secondaire est en outre divisé davantage et comprend entre 2 et 4 compartiments.

13. Utilisation d'un tube de prélèvement selon l'une quelconque des revendications précédentes, pour la collecte et l'échantillonnage de fluides corporels, par exemple d'urine.
